# EUROPEAN PATENT SPECIFICATION

(11) **EP 2 613 151 B1**
(45) Date of publication and mention of the grant of the patent: **29.06.2016**
(21) Application number: 11821292.7
(22) Date of filing: 26.08.2011
(51) Int. Cl.: G01N 33/574, G01N 21/64, G01N 33/48, G01N 33/53, G01N 33/536, G01N 33/58

(54) **METHOD FOR DETERMINING CANCER ONSET OR CANCER ONSET RISK**
VERFAHREN ZUR BESTIMMUNG DES AUSBRUCHS VON KREBS ODER DES RISIKOS FÜR DEN AUSBRUCH VON KREBS
MÉTHODE DE DÉTERMINATION DE L'APPARITION D'UN CANCER OU DU RISQUE D'APPARITION D'UN CANCER

(30) Priority: 02.09.2010 JP 2010196442
(43) Date of publication of application: 10.07.2013
(73) Proprietor: Tohoku University, Aoba-ku Sendai-shi Miyagi 980-8577 (JP)
(72) Inventor: GONDA, Kohsuke, Sendai-shi Miyagi 980-8577 (JP); MIYASHITA, Minoru, Sendai-shi Miyagi 980-8577 (JP); TAKEDA, Motohiro, Sendai-shi Miyagi 980-8577 (JP); OHUCHI, Noriaki, Sendai-shi Miyagi 980-8577 (JP)
(74) Representative: Appleyard Lees IP LLP
(86) International application number: PCT/JP2011/004762
(87) International publication number: WO 2012/029269

(56) References cited:
- WO-A1-2009/119455
- WO-A1-2010/023917
- WO-A1-2010/084720
- JP-A- 8 334 466
- JP-A- 2007 278 985
- JP-A- 2008 061 969
- JP-A- 2008 261 784
- JP-A- 2009 192 527
- P. CONSTANTINOU ET AL: "Extending immunofluorescence detection limits in whole paraffin-embedded formalin fixed tissues using hyperspectral confocal fluorescence imaging", JOURNAL OF MICROSCOPY, vol. 234, no. 2, 1 May 2009 (2009-05-01), pages 137-146, XP055107055, ISSN: 0022-2720, DOI: 10.1111/j.1365-2818.2009.03155.x
- C. H. VAN DE LEST ET AL: "Elimination of autofluorescence in immunofluorescence microscopy with digital image processing.", JOURNAL OF HISTOCHEMISTRY & CYTOCHEMISTRY, vol. 43, no. 7, 1 July 1995 (1995-07-01), pages 727-730, XP055107062, ISSN: 0022-1554, DOI: 10.1177/43.7.7608528
- HERNÃ NDEZ NORMA A ET AL: "PAR1 is selectively over expressed in high grade breast cancer patients: a cohort study", JOURNAL OF TRANSLATIONAL MEDICINE, BIOMED CENTRAL, LONDON, GB, vol. 7, no. 1, 18 June 2009 (2009-06-18), page 47, XP021059134, ISSN: 1479-5876, DOI: 10.1186/1479-5876-7-47
- KOHSUKE GONDA ET AL: "In Vivo Nano-imaging of Membrane Dynamics in Metastatic Tumor Cells Using Quantum Dots", JOURNAL OF BIOLOGICAL CHEMISTRY, AMERICAN SOCIETY FOR BIOCHEMISTRY AND MOLECULAR BIOLOGY, INC, BETHESDA, MD, USA, vol. 285, no. 4, 22 January 2010 (2010-01-22), pages 2750-2757, XP002653726, ISSN: 1083-351X, DOI: 10.1074/JBC.M109.075374 [retrieved on 2009-02-16]
- TAKEO ITO: 'Soshiki Saibo Kagaku 2006 -Idenshi - Bunshi kara Saibo - Soshiki eno Kaiki-, I. Men'eki Soshiki - Saibo Kagaku -Kiso kara Oyo- Keiko Kotai no Aratana Tenkai -Nano Crystal Men'eki Keiko Kotaiho' SOSHIKI SAIBO KAGAKU vol. 2006, 2006, pages 25 - 34, XP008168872
- YANG E. ET AL.: 'Blockade of PAR1 Signaling with Cell-Penetrating Pepducins Inhibits Akt Survival Pathways in Breast Cancer Cells and Suppresses Tumor Survival and Metastasis' CANCER RES vol. 69, no. 15, 2009, pages 6223 - 6231, XP055081238
- SHINAE KONDO ET AL.: 'Seitai Hikari Imaging o Mochiita Dobutsu Jikken' LABIO 21 no. 37, 2009, pages 14 - 18, XP008167970
- BARASH EUGENE ET AL.: 'Multiplexed Analysis of Proteins in Tissue Using Multispectral Fluorescence Imaging' IEEE TRANS MED IMAGING vol. 29, no. 8, August 2010, pages 1457 - 1462, XP011305275

## Description

### Technical Field

The present invention relates to a method for determining cancer onset or cancer onset risk which uses a highly accurate, quantitatively analyzable tissue staining method from which the influence of autofluorescence is effectively eliminated.

### Background Art

Cancer is a disease which splits the causes of adult death with vascular diseases represented by myocardial infarction and cerebral infarction. For example, the breast cancer incidence rate in Japanese is lower than those in European and American countries, but it tends to increase in recent years and has ranked No. 1 in the female incidence rate overtaking the incidence rate of stomach cancer in 1998. According to a recent report, the year 2005 statistics projected by Ministry of Health, Labor and Welfare, the annual incidence number of breast cancer exceeds 50000 cases.

Cancer diagnosis commonly uses, in addition to image diagnoses such as X-ray CT and MRI, methods for detecting a cancer marker which is specifically expressed to a certain cancer or a cancer marker which leaks out in blood or tissues. The typical breast cancer screening test includes an interview, palpation, soft X-ray mammography (mammography) or ultrasonography examination and, when clinically suspected, cytodiagnosis or biopsy is performed to determine whether it is a breast cancer or not based on the pathological diagnosis. The pathological diagnosis is important to assess cancer treatment and prognostic conditions, and the leading diagnoses are "HE staining method for conducting the structure observation" and "immunohistochemical method using an antibody to a cancer marker factor". Particularly, owing to the antibody drugs developed in recent years, the immunohistochemistry has become far more important.

For example, Herceptin (HER2 antibody: the antibody to Human Epidermal Growth Factor Receptor 2) (registered trademark) is a representative example of the anti-cancer drug for breast cancers, and the immunohistochemical method using the HER2 antibody is clinically carried out as the criterion for the administration of this drug. However, the conventional methods by the immunohistochemical method have been performed by visual diagnosis by a pathologist using an enzyme method represented by DAB. For this reason, the detection sensitivity was low and quantitativity was problematic.

Recently, fluorescent particles such as quantum dot have drawn attentions as detection methods with good detection sensitivity and quantitativity instead of the DAB enzyme method. Quantum dot has the number of particles and the fluorescent brightness in the proportional relationship, and thus has a potential for quantitatively analyzing an expression level of a cancer marker with high accuracy when a probe in which quantum dots are conjugated to an antibody of a cancer marker is used for the immunohistochemical method.

Metastasis is one of the primary threats in cancers. PAR1 (protease activated receptor 1) is a membrane protein which activates the mobility and infiltration ability of cancer cells, and is known to have been associated with the metastasis of a wide variety of cancers (breast cancers, lung cancers, pancreatic cancers, prostate cancers and the like). Particularly, in breast cancers, PAR1 is expressed in most of the cancer cell lines with metastatic ability, and it is considered that when the N-terminal extracellular region of PAR1 is cleaved by matrix metalloprotease 1 (MMP1: Matrix Metalloprotease 1), PAR1 is activated whereby the cell mobility and infiltration ability are enhanced (see for example, non-patent document 1). The present inventors have documented that a PAR1 antibody, which inhibits the cleavage of PAR1 by MMP1 and thus inhibits the cancer cell mobility and infiltration ability, is produced and such an antibody inhibits the cell mobility activity and cell infiltration activity induced by MMP1 (see for example, patent document 1).

### Prior Art Documents

### Patent Documents

Patent Document 1: International Publication No. 09/119455

### Non-patent Documents

Non-patent Document 1: Boire A, Covic L, Agarwal A, Jacques S, Sherifi S, Kuliopulos A. PAR1 is a matrix metalloprotease-1 receptor that promotes invasion and tumorigenesis of breast cancer cells. Cell 120:303-313
(2005) Non-patent Document 2: P. Constantinou et al, J. Microsc. 2009, 234, 137-146, describes extending immunofluorescence detection limits in whole paraffin-embedded formalin fixed tissues using hyperspectral confocal fluorescence imaging. Non-patent Document 3: C. H. Van de Lest et al, J. Histochem. Cytochem. 1995, 43, 727-730 describes the elimination of autofluorescence in immunofluorescence microscopy with digital image processing.

### Summary of the Invention

### Object to be Solved by the Invention

Fluorescent particles such as quantum dot exhibit a potential for the intrinsic light stability and quantitative analysis, and is thus highly expected as a new tool for the fluorescent immunostaining but, for the quantitative analysis, it is still limited to cultured cells. The major reason for this limit is because that the fluorescent brightness of quantum dot fluorescent particles can be calculated as a sufficiently high S (signal)/N (noise) ratio due to the weak cell autofluorescence in cultured cells, whereas, cells in biological tissues have extremely stronger autofluorescence than those in cultured cells and the fluorescent brightness of quantum dot fluorescent particles cannot be calculated as a sufficiently high S/N ratio. The autofluorescence of the long wavelength is a smaller amount than the autofluorescence of the short wavelength in biological tissues, but the influence thereof to the observation is not negligible and thus the quantitative analysis of quantum dot fluorescent particles in biological tissues was difficult. An object of the present invention is to provide a highly accurate and quantitative method for determining cancer onset or cancer onset risk by the quantitative tissue staining method in biological tissues using an antibody recognizing a cancer growth regulatory factor or metastasis regulatory factor such as PAR1 antibody which inhibits the mobility and infiltration ability of cancer cells.

### Means to Solve the Object

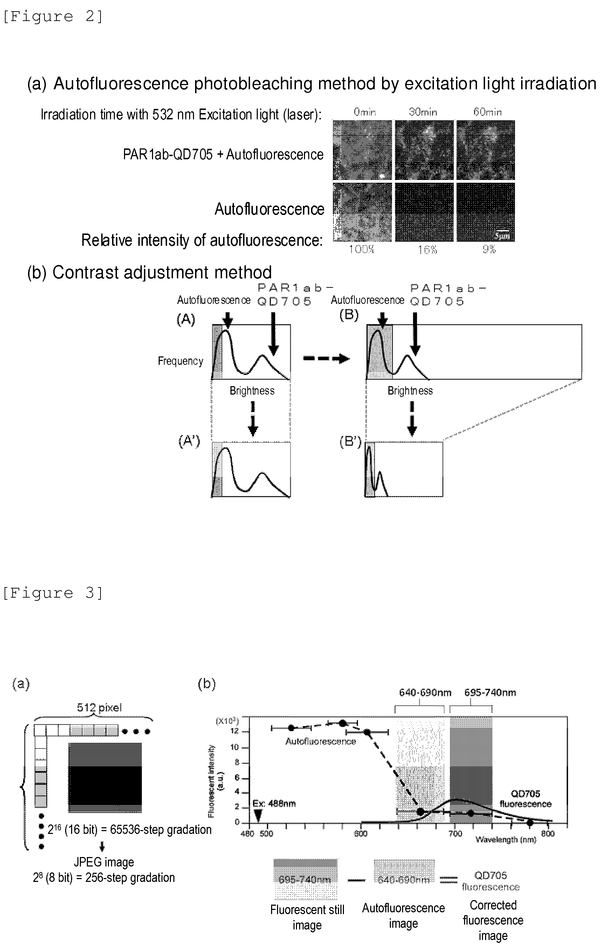

(a) Autofluorescence photobleaching method by excitation light irradiation, (b) contrast adjustment method and the like are known as methods for eliminating the influence of autofluorescence to the biological tissue staining method (see Figure 2). In the above (a) autofluorescence photobleaching method by the excitation light irradiation, it is possible to bleach only the autofluorescence while the brightness intrinsic to quantum dot fluorescent particles is left unbleached by light irradiation for an extended period of time using the light stability of quantum dot fluorescent particles, however, it poses drawbacks in that the complete bleaching requires many hours and only autofluorescence on an irradiated area is bleached. Further, the above (b) contrast adjustment method has drawbacks in that, when the fluorescent brightness of quantum dot fluorescent particles is weaker than the brightness of autofluorescence, the fluorescent brightness of quantum dot fluorescent particles diminishes by the contrast adjustment to erase the autofluorescence image, and further a definite threshold cannot be determined because the autofluorescence widely varies depending on the type of and place in a sample. The present inventors conducted extensive studies on methods for eliminating the influence of autofluorescence in place of the above methods (a) and (b), and found that, after acquiring a fluorescence image of quantum dot fluorescent particles (fluorescence of quantum dot fluorescent particles + autofluorescence) by irradiating a breast cancer tissue sample, which is immunostained with a PAR1 antibody (PAR1ab-QD705) labeled with a quantum dot fluorescent particle (Qdot705), with excitation light having a wavelength of 488 nm through a band pass filter having an acquisition region of fluorescence wavelength of 695 to 740 nm, an autofluorescence image showing only autofluorescence is acquired in the exactly same field of vision and the same focal plane as those of the fluorescence image through a band pass filter having an acquisition region of fluorescence wavelength of 640 to 690 nm, followed by subtracting the corresponding fluorescent brightness of each pixel in the autofluorescence image from the fluorescent brightness of each pixel of the fluorescent still image, whereby an image showing only the fluorescence of quantum dot fluorescent particles (corrected fluorescence image), in which the autofluorescence image (autofluorescence) is subtracted from the fluorescent still image, can be acquired. Further, the "blinking property" distinctive of the quantum dot is used to specify the particle number of quantum dot fluorescent particles included in the corrected fluorescence image. In other words, it is confirmed that a single quantum dot fluorescent particle has about 4 seconds of off-state in excitation light irradiation time of 20 seconds, based on which the mean brightness of the particles having an about 4 second off-state is calculated to be the fluorescent brightness of a single quantum dot fluorescent particle, whereby the number of quantum dot fluorescent particles per cell in the corrected fluorescence image can be calculated. Furthermore, using tissues of recurrence-free breast cancer survivors, breast cancer tissues of recurrent patients and normal mammary tissues of cancer patients, the numbers of quantum dot fluorescent particles detected with PAR1ab-QD705 were calculated, and it was revealed that the numbers of quantum dot fluorescent particles detected with PAR1ab-QD705 were significantly higher in the tissues of recurrence-free breast cancer survivors and breast cancer tissues of recurrent patients than those in the normal mammary tissues of cancer patients. The present invention has been accomplished based on these findings.

More specifically, the present invention relates to: (1) a method for determining cancer onset or cancer onset risk, comprising using a tissue staining method comprising the steps of: (a) labeling an antibody which recognizes a cancer growth regulatory factor or cancer metastasis regulatory factor with a fluorescent material, and contacting the fluorescent-labeled antibody with a tissue sample; (b) irradiating a tissue site in contact with the antibody with excitation light of a given wavelength; (c1) acquiring from step (b) a fluorescence image in an acquisition region of fluorescence wavelength emitted by the fluorescent material; (c2) acquiring from step (b) an autofluorescence image in a vicinity region of a short wavelength side or long wavelength side of an acquisition region of fluorescence wavelength emitted by the fluorescent material, in the same field of vision and in the same focal point as those of the fluorescence image; (d) acquiring a corrected fluorescence image by image processing to eliminate a fluorescent brightness of the autofluorescence image from the fluorescent brightness of the fluorescence image; (2) the determination method according to the above (1), wherein the cancer growth regulatory factor or cancer metastasis regulatory factor is PAR1 (protease activated receptor 1); (3) the determination method according to the above (1) or (2), wherein the cancer is a breast cancer; (4) the determination method according to any one of the above (1) to (3), wherein a difference in the wavelengths between the acquisition region of fluorescence wavelength emitted by the fluorescent material and the vicinity region thereof is within 100 nm; (5) the determination method according to any one of the above (1) to (4), wherein the acquisition region of fluorescence wavelength emitted by the fluorescent material is a near infrared region; (6) the determination method according to any one of the above (1) to (5), wherein the excitation wavelength of the excitation light is 400 to 700 nm; (7) the determination method according to any one of the above (1) to (6), wherein the fluorescent material is a fluorescent particle; (8) the determination method according to the above (7), wherein the fluorescent particle is a quantum dot fluorescent particle; (9) the determination method according to the above (7) or (8), wherein the tissue staining method further comprises (e) counting the number of cells at the tissue site in contact with the antibody; (f) identifying a single fluorescent particle based on the fluorescence image, and measuring a mean fluorescent brightness of a single fluorescent particle in the corrected fluorescence image corresponding to the single fluorescent particle; and (g) calculating the number of fluorescent particles per cell by dividing a total fluorescent brightness in the corrected fluorescence image by the mean fluorescent brightness of the single fluorescent particle to determine the number of fluorescent particles, and dividing the number of fluorescent particles by the number of cells counted in the step (e); and (10) the determination method according to the above (9), wherein the single fluorescent particle is specified using a blinking property of the fluorescent particles.

### Effect of the Invention

According to the tissue staining method of the present invention wherein the influence of autofluorescence is eliminated and the PAR1 antibody labeled with quantum dot fluorescent particles is used, the average value of the number of quantum dot fluorescent particles per cell in mammary tissues (detected using the PAR1 antibody) was 0.2 in 4 samples of normal mammary tissues from cancer patients, whereas 3.2 in 5 samples of tissues from recurrence-free breast cancer survivors for at least 5 years and who are HercepTest negative (detected using the HER2 antibody) and 14.8 in 3 samples of breast cancer tissues from recurrent patients (recurred within about 1 year and died within 4 years) who are HercepTest negative (detected using the HER2 antibody), thus acquiring significant differences. Further, "triple negatives", which refers to any breast cancer that develops with no connection to three major breast cancer growth-related factors [estrogen receptor (ER; estrogen receptor), progesterone receptor (PgR; progesterone receptor), and human epidermal growth factor receptor 2 (HER2: human epidermal growth factor receptor 2)], has been reported, and "triple negative" is a serious issue because HercepTest is not valid, but the average value of the number of quantum dot fluorescent particles per cell (detected using the PAR1 antibody) was 6.0 in 8 samples of "triple negative" breast cancer tissues, obtaining a significant difference in comparison with the normal mammary tissues of cancer patients. Using this tissue staining method, quantitative breast cancer pathological diagnosis with high sensitivity has become viable for breast cancer patients who are even determined to be HercepTest negative. Thus, according to the present invention, not only cancer recurrence or cancer recurrence risk can be determined but cancer onset or cancer onset risk can also be determined.

### Brief Description of Drawings

[Figure 1] Figure 1(a) shows the preparation process of an immunostained tissue sample. Figure 1(b) shows the result of fluorescence microscopic analysis on an immunostained tissue sample. The white dotted lines represent the outlines of cancer cells. The white arrows indicate the fluorescence of quantum dot fluorescent particles (detected using PAR1ab-QD705) and the white arrowheads indicate the fluorescence of autofluorescence.
[Figure 2] Figure 2 illustrates the conventional methods for eliminating the influence of autofluorescence. Figure 2(a) shows the results of autofluorescence photobleaching method (see Hikage et al. Nanotechnology (2010)) by an excitation light irradiation, and Figure 2(b) illustrates the contrast adjustment method. The gray-shaded zones shown in (b) represent the areas that look black with human eyes.
[Figure 3] Figure 3(a) shows an image conversion in the image processing by the subtraction method. Figure 3(b) shows the fluorescence wavelength behavior of quantum dot fluorescent particles and the fluorescence wavelength behavior of autofluorescence. The solid line represents the fluorescence wavelength behavior of quantum dot fluorescent particles (QD705). The plots with error bars represent the measured values of fluorescent brightness of the autofluorescence on tissue sample sections acquired respectively through 6 types of band pass filters in total having an acquisition region of fluorescence wavelength of 505 to 545 nm, 565 to 595 nm, 585 to 630 nm, 640 to 690 nm, 695 to 740 nm and 760 to 800 nm. The average value plots of these measured values are connected with a dotted line to represent the fluorescence wavelength behavior of autofluorescence. The wavelength width of band pass filters is represented by a bar.
[Figure 4] Figure 4 shows the method for producing a corrected fluorescence image from which autofluorescence is eliminated.
[Figure 5] Figure 5 shows the results of an acquired corrected fluorescence image from which autofluorescence is eliminated, using a tissue sample in which cancer cell tissues and normal cell tissues are present close to each other.
[Figure 6] Figure 6 shows the blinking property of quantum dot fluorescent particles. The ordinate represents the fluorescent brightness of quantum dot fluorescent particles and the abscissa represents the irradiation time (second) of quantum dot fluorescent particles with excitation light. The arrowhead indicates that 10% of the maximum fluorescent brightness of quantum dot fluorescent particles is defined as the threshold of fluorescent brightness.

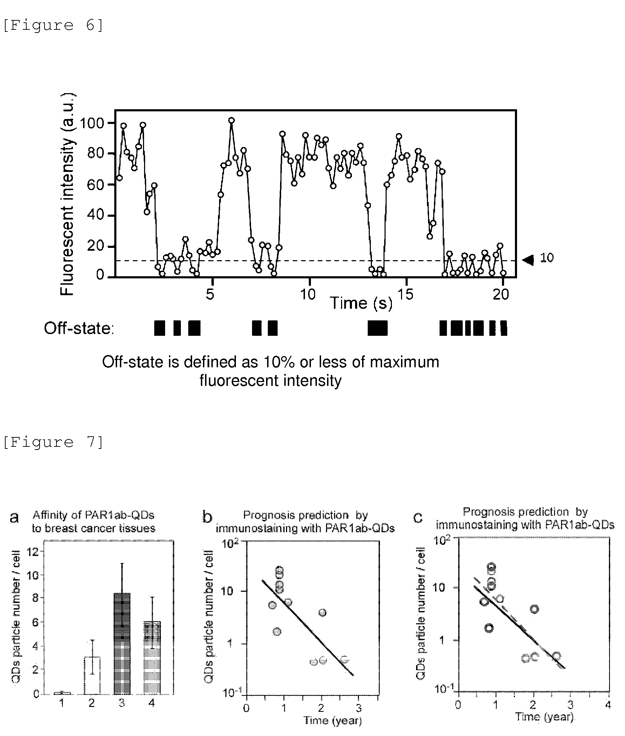

[Figure 7] Figure 7(a) shows the calculation results of the true number of particles per cell (represented by the ordinate) detected using PAR1 antibody. 1 to 4 on the abscissa represent respectively 1: normal mammary tissues from cancer patients, 2: tissue from recurrence-free breast cancer survivors for at least 5 years, 3: breast cancer tissues from recurrent patients (cases of recurrence within 4 years and death within 6 years), 4: breast cancer tissues from "triple negative" recurrent patients (cases of recurrence within 4 years · death within 6 years). Figure 7(b) shows the relation between the true particle number per cell detected with the PAR1 antibody (represented by the ordinate) and time in years to breast cancer recurrence (represented by the abscissa). Figure 7(c) shows the result of breast cancer tissues from "triple negative" recurrent patients in the data of Figure 7(b). For reference, the results of breast cancer tissues from recurrent patients other than "triple negative" patients are shown in light color o.
[Figure 8] Figure 8 shows the comparison results of 488 nm and 532 nm excitation wavelengths of excitation light.
[Figure 9] Figure 9 shows the examination results on the wavelength ranges of band pass filters for acquiring an autofluorescence image under the condition of 488 nm excitation wavelength of excitation light.
[Figure 10] Figure 10 shows the examination results on the wavelength ranges of the band pass filters for acquiring an autofluorescence image under the condition of 532 nm excitation wavelength of excitation light.
[Figure 11] Figure 11 shows the results of DAB stained cancer tissue samples, of which the numbers of quantum dot fluorescent particles were calculated.

### Mode of Carrying Out the Invention

The method for determining cancer onset or cancer onset risk of the present invention is not particularly limited insofar as the method comprises using the tissue staining method (with the proviso that diagnosis by a doctor is excluded) comprising the steps of: (a) labeling an antibody which recognizes a cancer growth regulatory factor or cancer metastasis regulatory factor with a fluorescent material, and contacting the fluorescent-labeled antibody with a tissue sample; (b) irradiating a tissue site in contact with the antibody with excitation light of a given wavelength; (c1) acquiring from step (b) a fluorescence image in an acquisition region of fluorescence wavelength emitted by the fluorescent material; (c2) acquiring from step (b) an autofluorescence image in the vicinity region of the short wavelength side or long wavelength side of an acquisition region of fluorescence wavelength emitted by the fluorescent material, in the same field of vision and in the same focal point as those of the fluorescence image; (d) acquiring a corrected fluorescence image by image processing to eliminate a fluorescent brightness of the autofluorescence image from the fluorescent brightness of the fluorescence image, and the tissue staining method preferably further comprises (e) counting the number of cells at the tissue site in contact with the antibody; (f) identifying a single fluorescent particle based on the fluorescence image, and measuring a mean fluorescent brightness of a single fluorescent particle in the corrected fluorescence image corresponding to the single fluorescent particle; and (g) calculating the number of fluorescent particles per cell by dividing a total fluorescent brightness in the corrected fluorescence image by the mean fluorescent brightness of the single fluorescent particle to determine the number of fluorescent particles, and dividing the number of fluorescent particles by the number of cells counted in the step (e).

Examples of the cancer growth regulatory factor of the cancer growth regulatory factor or cancer metastasis regulatory factor in the step (a) may include factors which regulate the cell growth such as epidermal growth factor (EGF), EGF receptor (EGFR), platelet-derived growth factor (PDGF), PDGF receptor (PDGFR), insulin-like growth factor (IGF), IGF receptor (IGFR), fibroblast growth factor (FGF), FGF receptor (FGFR), vascular endotherial growth factor (VEGF), VEGF receptor (VEGFR), hepatocyte growth factor (HGF), HGF receptor (HGFR), neurotropin (NT), transforming growth factor-β (TGFβ) family, human epidermal growth factor receptor 1, 2, 3 or 4 (HER1, 2, 3 or 4), macrophage colony-stimulating factor 1 (CSF1) and CSF1 receptor (CSF1R), or factors which regulate the cell cycle such as cyclin, cyclin-dependent kinase (CDK), cyclin A, cyclin B, cyclin D, cyclin E, CDK1, CDK2, CDK4, CDK6, p16INK, p15, p21, p27, RB (retinoblastoma), and examples of the cancer metastasis regulatory factor may include Matrix Metalloprotease 1 (MMP1), Matrix Metalloprotease 2 (MMP2), PAR1 (protease activated receptor 1), CXCR4 (chemokine[C-X-C motif] receptor 4), and CCR7(chemokine [C-C motif] receptor 7), with PAR1 being preferable thereamong.

The antibody which recognizes a cancer growth regulatory factor or cancer metastasis regulatory factor in the above step (a) is preferably an antibody which specifically recognizes a growth regulatory factor or metastasis regulatory factor, and examples of the type of antibody may include the monoclonal antibody and polyclonal antibody. Class or subclass of the above antibodies are not particularly limited, and examples of the class may include IgA, IgG, IgE, IgD and IgM, and examples of the subclass may include IgG1, IgG2, IgG3, IgG4, IgA1 and IgA2. The term "antibody" used herein means to encompass any antibody fragments or derivatives, and examples thereof include Fab, Fab'₂, CDR, humanized antibodies, multifunctional antibodies and single-chain variable fragments (ScFv). These antibodies can be produced by known methods (see for example, Harlow E. & Lane D., Antibody, Cold Spring Harbor Laboratory Press (1988)) but commercial products may also be used. Examples of the antibody which recognizes the above PAR1 may include a commercial PAR1 antibody which recognizes the N-terminal region of PAR1 and the PAR1 antibody disclosed in International Publication No. 09/119455.

The fluorescent material in the above step (a) is not particularly limited insofar as the substance emits fluorescent in response to the irradiation with radiations of a selected wavelength such as ultraviolet rays and visible light, and examples of the organic fluorescent material may include fluorescein, rhodamine, Cy-5, Cy-3 and alexa, and examples of the inorganic fluorescent material may include fluorescent particles such as fluorescent silica nanoparticle and quantum dot fluorescent particle. Further, since the influence of autofluorescence is smaller on the long wavelength side, a fluorescent material which emits fluorescence on the near infrared region side, particularly, of the near infrared region is used, and it is preferable that the acquisition region of the fluorescence wavelength emitted by the fluorescent material be the near infrared region side (570 to 800 nm), particularly the near infrared region (700 to 800 nm). The above organic fluorescent materials quickly bleach by excitation light and are hence not suitable for the analysis requiring irradiation with excitation light for an extended period of time. For this reason, it is preferable to use fluorescent particles with good light stability which are thus stable even when irradiated with excitation light for an extended period of time, with quantum dot fluorescent particles being preferable thereamong. The fluorescent particle may be those which emit fluorescence when irradiated with excitation light, and the material thereof or the like is not limited and may be materials which emit fluorescence themselves or particles which contains fluorescent materials or coated with fluorescent materials so as to emit fluorescence. The shape and size of fluorescent particle are not particularly limited and examples thereof may include rectangle, disc form, polyhedron and spherical, but spherical is preferable and the size thereof has a diameter of 0.00001 nm to 1 mm, preferably a diameter of 0.001 nm to 100 µm, further preferably a diameter of 0.01 nm to 10 µm, and the wavelength of fluorescence emitted by the fluorescent particles can be suitably selected. The fluorescent particle can be produced by the methods disclosed in Japanese unexamined Patent Application Publication No. 9-241634 and Japanese unexamined Patent Application Publication No. 2010-242059, but commercial products may be purchased such as SPHERO fluorescent particle (a product of Bay biosciece Inc.) prepared by staining polystyrene particles in the presence of polystyrene core particle using the polymerization of a suitable fluorescent dye or styrene fluorescent dye or fluorescent particle Estapor (registered trademark) standard microparticle (a product of Merck Chime S.A.S.). Among the fluorescent particles, quantum dot fluorescent particle, also called colloidal quantum dot (QD: quantum Dot), characteristically has extremely intense emission spectrum together with high quantum efficiency, hence preferable. The quantum dot fluorescent particle is a fluorescent semiconductor nanoparticle with a diameter ranging from 1 to 20 nm, and thus has been used for fluorescence imaging in the field of biology and medical diagnosis. In a quantum dot fluorescent particle, electrons are quantumly confined in a clear-outlined 3D nano size semiconductor crystal, and since a smaller size quantum dot fluorescent particle has a wider semiconductor band gap, it is possible to adjust semiconductor photoluminescence emission wavelength throughout the visible spectrum in accordance with the size. The quantum dot fluorescent particle may be luminescent semiconductor nanoparticles, and it is desirable to suitably adjust the particle size thereof based on the excitation light and fluorescence wavelength. Examples of the quantum dot fluorescent particle which can be used may include nanocrystals of a semiconductive material (CdSe) coated with a semiconductor shell (ZnS) such as Qdot (registered trademark)525, Qdot565, Qdot585, Qdot605, Qdot625, Qdot655, Qdot705 and Qdot800 (all products of Invitrogen). When a fluorescence wavelength to be acquired exceeds 800 nm, a camera sensitivity is reduced, and it is thus preferable to use, among quantum dot fluorescent particles emitting fluorescence of the near infrared region, the quantum dot fluorescent particle (Qdot705) which emits the 705 nm fluorescence.

In the above step (a), examples of the method for labeling the antibody with a fluorescent material may include a method which mediates an antibody (secondary antibody) having a specific affinity to the antibody, the biotin-avidin method, the coupling reaction of thiol group-maleimide group, a method which uses an existing chemical linker, a crosslinking reaction which uses a crosslinking agent (EDC, etc.) and ionic bonds. For examples, the antibody and quantum dot fluorescent particles are bonded in accordance with an instruction manual of a manufacturer, using Qdot Antibody Conjugation Kit (Invitrogen), etc.

Examples of the tissue sample in the above step (a) may include fixed tissue sample sections such as formalin-fixed paraffin sections and frozen sections; pathological tissue samples, peripheral blood tissue samples, cell block samples of cells isolated from tissues or cells isolated from body fluids, cell block samples prepared by precipitating or centrifuging floating samples of peripheral blood leukocytes or cells, imprint samples of tissues, and liquid smear samples containing peripheral bloods, body fluids, exudates or cells.

In the above step (a), the method for contacting the fluorescent-labeled antibody with a tissue sample is not particularly limited, but the temperature at the time of contact is preferably 0 to 40 °C since the contact induces the antigen-antibody reaction. The contact time varies depending on the type of tissue sample to be used, an antibody concentration, a titer height, the above contact (reaction) temperature, a level of target factor (antigen), the location and the like, and is suitably selected without particular limitation, but examples thereof may include 10 minutes to 12 hours, preferably 10 minutes to 2 hours. Further, to prevent the nonspecific bonding of the antibody, the blocking treatment using FBS, BSA, IgG or the like can be performed before the antibody is contacted with a tissue sample.

Specific examples of the excitation wavelength of excitation light in the above step (b) may include, in view of being a shorter wavelength than the fluorescence wavelength emitted by the fluorescent material and safety to the human body, preferably 400 to 700 nm, more preferably 450 to 650 nm, and specifically 488 nm, 532 nm or 635 nm used for typical excitation wavelength.

Examples of the fluorescence image in the above step (c1) may include a fluorescent still image, a fluorescence videograph corresponding to a fluorescent still image and the fluorescent still image. The above fluorescent still image can be acquired using a computer such as a common PC as the controller and a fluorescence microscope which can, for example, regulate photographing conditions of a CCD camera, image and display a captured fluorescent still image and control a light intensity from a light source. Necessary settings such as the filter and dichroic mirrors on the excitation light side are done so that a fluorescent brightness emitted by the fluorescent material does not saturate, and it is further preferable that the exposure time of excitation light be set to be ranging between 0.0003 and 30 to 60 seconds. For the method to check out if the fluorescent brightness of each pixel of the fluorescent material is saturated or not, a brightness value is prespecified or a brightness value at a prespecified ratio to the maximum intensity value (for example, for an 8-bit image having the maximum value of 255, a numerical value of 220 or 90% of the maximum brightness) is decided and the saturation may be confirmed when this value is exceeded. Alternatively, the saturation may also be confirmed using a plurality of pixels at a prespecified ratio to the entire image instead of a single pixel. When the fluorescent brightness of the fluorescent material in a fluorescent still image is specified whether it is derived from a single particle or not, a fluorescence videograph can be acquired as necessary. For the above fluorescence videograph, it is preferable to acquire 50 to 200 frames of the videograph in the same field of vision and the same focal point as those of the above fluorescent still image at a resolution of 2 to 500 msec. For the band pass filter for acquiring the above fluorescence image, it is desirable to use a band pass filter which selectively allows only fluorescence of specific wavelengths emitted by the fluorescent material such as red, cyan, orange, green or blue to penetrate and is capable of acquiring 25% or more, preferably 50% or more, more preferably 75% or more, of the total fluorescent brightness of the above fluorescent material. Further, for the band pass filter for acquiring the above fluorescence image, it is desirable to use a band pass filter which can acquire 30% of the fluorescent brightness of the above fluorescent material in a wavelength range of within 30 nm, preferably within 15 nm, more preferably within 5 nm. For example, when a fluorescent material emitting the 705 nm fluorescence is used, the band pass filter for acquiring the above fluorescence image is preferably a band pass filter having an acquisition region of fluorescence wavelength of 695 to 740 nm.

Examples of the method for acquiring an autofluorescence image in the above step (c2) may include a method wherein an image is acquired in the same field of vision and in the same focal point conditions as those of the above fluorescent still image using, in place of the band pass filter used to acquire the fluorescence wavelength emitted by the fluorescent material, a band pass filter which can acquire a wavelength in the vicinity region of the short wavelength side or long wavelength side of the band pass filter, preferably a wavelength in the vicinity region of the short wavelength side, whereby an image containing only the autofluorescence without including the fluorescence of the fluorescent material is acquired. The fluorescence of long wavelength has a smaller refraction than the fluorescence of short wavelength and is focused farther than the fluorescence of short wavelength. For this reason, when the acquisition region of fluorescence wavelength emitted by the above fluorescent material is largely different from the wavelength of wavelength range for acquiring an autofluorescence image, a wide gap is resulted between the focal point of autofluorescence included in the above fluorescent still image and the focal point of autofluorescence of the autofluorescence image. For this reason, preferable examples of the difference in wavelengths between the acquisition region of fluorescence wavelength emitted by the fluorescent material and the vicinity region thereof may include regions of 130 nm or less, preferably 120 nm or less, more preferably 110 nm or less, and particularly 100 nm or less. Examples of the band pass filter used to acquire the fluorescence image and autofluorescence image may include those having an acquisition region of fluorescence wavelength of specifically 505 to 545 nm, 565 to 595 nm, 585 to 630 nm, 640 to 690 nm, 695 to 740 nm or 760 to 800 nm, and, for example, when the above fluorescent still image is acquired using a band pass filter having an acquisition region of fluorescence wavelength of 695 to 740 nm, a band pass filter for acquiring an autofluorescence image is preferably a band pass filter having an acquisition region of fluorescence wavelength of 640 to 690 nm. For the photographing conditions to acquire the above autofluorescence image, the brightnesses of autofluorescence in the same region of the above fluorescent still image and the autofluorescence image are compared and any ROI may be set, for example, the maximum value of the autofluorescence having 25 x 25 pixels in the autofluorescence image becomes 1.1 to 1.3 times, preferably 1.2 times, of the fluorescent still image. More specifically, it is preferable that the autofluorescence image has a 10 to 30%, particularly about 20%, higher brightness value of autofluorescence than that of a fluorescent still image.

Examples of the method for acquiring a corrected fluorescence image in the above step (d) may include a process wherein an fluorescent still image and autofluorescence image are converted to an image file such as JPEG or TIF, and then the brightness of the autofluorescence is eliminated from the brightness of the fluorescent still image based on the brightness of the autofluorescence image, using an image analysis software such as Image·J (http://rsb.info.nih.gov/ij/), Photoshop (a product of Adobe Systems Incorporated), After Effect (a product of Adobe Systems Incorporated) or G-Count (a product of G-Angstrom K.K.). In addition, at the time of converting to an image file, it is preferable to select a threshold to include all fluorescence distributions.

Examples of the method for counting the number of cells at the tissue site which was in contact with the antibody in the above step (e) may include a method wherein cell nucleus are stained with a nucleic acid staining agent which emits fluorescence when bonded to DNA such as DAPI, Hoechst or PI (Propidium Iodide), and then the total number of cell nucleus stained in the same field of vision as those of the fluorescence image is counted; however, when the acquisition region of fluorescence wavelength emitted by the fluorescent material is a near infrared region, it is more preferable to use DAPI or Hoechst which emits blue fluorescence than PI which emits red fluorescence whose fluorescence property is similar thereto.

Examples of the method for identifying a single fluorescent particle in the above step (f) may include a method wherein a fluorescent brightness of the fluorescent particles in the fluorescence image is measured and, based on the brightness information of the fluorescence emitted by a single fluorescent particle, the number of particles to which the above fluorescent particles correspond is determined or a method wherein the "blinking property" distinctive of the fluorescent particles is used; however, when the fluorescent particle is the quantum dot fluorescent particle, the method which uses the blinking property is preferable. The "blinking property" used herein refers to the repetitious property that the off time (fading time), during which an emission intensity suddenly diminishes substantially to zero (off) to an irradiation time or an irradiation level when irradiated with excitation light, lasts for almost several msec to 5 sec and the previous emission intensity comes back again. Further, the method for identifying a single fluorescent particle which uses the "blinking property" refers to a method for identifying a single fluorescent particle wherein the fading time of a fluorescent particle is measured when irradiated with excitation light for any time of period, for example, 10 to 100 seconds, based on the fluorescent still image or fluorescence videograph, and the fluorescent particle is evaluated whether it is a single particle or not. For example, when the quantum dot fluorescent particle emitting the 705 nm fluorescence is used as the fluorescent particle, the fading time of a single quantum dot fluorescent particle when irradiated with excitation light for 20 seconds is about 4 seconds and thus a fluorescent particle having a fading time of about 4 seconds can be specified to be the quantum dot fluorescent particle derived from a single particle. To determine if the quantum dot fluorescent particle faded or not, the fluorescent brightness threshold of quantum dot fluorescent particle can be suitably determined, and, for example, 0 to 30%, preferably 5 to 15%, more preferably 10%, of the maximum fluorescent brightness of the quantum dot fluorescent particle is set as the threshold. The fluorescent brightness in the corrected fluorescence image corresponding to the specified single particle is measured using an image analysis software such as Image·J, Photoshop, After Effect or G-Count. The calculation of mean fluorescent brightness of a single particle by the above measurement is carried out using 2 to 200 fluorescent particles, preferably 5 to 10 fluorescent particles, in consideration of labor and accuracy.

In the above step (g), the number of fluorescent particles per cell is calculated. The number of fluorescent particles per cell can be calculated by inputting the value of total fluorescent brightness of the quantum dot fluorescent particles acquired from the above corrected fluorescence image (the total fluorescent brightness in the image), the number of cells acquired from the above cell nucleus stained image and the value of mean fluorescent brightness per quantum dot fluorescent particle (a single particle fluorescent brightness) into the formula [(the total fluorescent brightness in the image / the single particle fluorescent brightness) / the number of cells = the number of total fluorescent particles in the image / the number of cells = the number of fluorescent particles / cell].

According to the determination method of the present invention comprising the above steps (a) to (d), corrected fluorescence images of a tissue sample to be determined and a normal tissue sample are respectively acquired, the fluorescent brightnesses in both corrected fluorescence images are compared, and when the fluorescent brightness in the corrected fluorescence image of the tissue sample to be determined is higher, a protein amount of the cancer growth regulatory factor or metastasis regulatory factor of the tissue sample to be determined is suggested to be large, whereby the tissue to be determined can be determined to have cancer onset or a cancer onset risk. Further, according to the determination method of the present invention comprising the above steps (a) to (g), the numbers of fluorescent particles per cell in a tissue sample to be determined and in a normal tissue sample are respectively calculated, both numbers are compared, and when the number of fluorescent particles per cell in the tissue sample to be determined is larger, a protein amount of the cancer growth regulatory factor or metastasis regulatory factor of the tissue sample to be determined is suggested to be large, whereby the tissue to be determined can be determined to have cancer onset or a cancer onset risk. For example, the antiPAR1 antibody, which detects a breast cancer marker factor, is labeled with quantum dot fluorescent particles, using such a quantum dot fluorescent particle labeled antibody, the number of fluorescent particles per cell detected using the antiPAR1 antibody is calculated and the pathological diagnosis of breast cancer onset or a breast cancer onset risk can be performed using the calculated number as the index.

The present invention may be carried out using (11) a system for determining cancer onset or cancer onset risk comprising: (A) an antibody which recognizes a cancer growth regulatory factor or cancer metastasis regulatory factor labeled with a fluorescent material; (B) excitation light irradiation means; (C) fluorescence image acquisition means; and (D) a band pass filter for acquiring a fluorescence image; (12) the determination system according to the above (11), wherein the antibody which recognizes a cancer growth regulatory factor or cancer metastasis regulatory factor is PAR1 (protease activated receptor 1); (13) the determination system according to the above (11) or (12), further comprising (E) a band pass filter for acquiring a cell nucleus fluorescence image; (14) the determination system according to any one of the above (11) to (13), wherein the fluorescent material is a fluorescent particle; and (15) the determination system according to the above (14), wherein the fluorescent particle is a quantum dot fluorescent particle.

Examples of an excitation light irradiation means in the determination system which may be used to carry out the present invention include mercury lamp (100 V), mercury lamp (200 V), xenon lamp (75 V), xenon lamp (150 V), halogen lamp (12 V 100 W), tungsten lamp (6 V 30 W), xenon lamp [wavelength 250 to 1,000 nm], tungsten lamp [wavelength 250 to 1,000 nm], Cr: LiSAF lamp [wavelength 430 nm], helium-cadmium laser [wavelengths 325, 442 nm], UV argon laser [wavelengths 351, 364 nm], argon ion laser [wavelengths 488, 514 nm], helium neon laser [wavelengths 543, 594, 633 nm], Krypton ion laser [wavelengths 568, 647 nm], and LD excitation CW/Q-CW (continuation oscillation / semi-continuation oscillation) solid-state laser [wavelengths 375 nm, 405, 440, 473, 488, 505, 515, 532, 561, 594, 635, 785 or 1064 nm], and, of these, preferable examples may include excitation wavelength of 400 to 700 nm, preferably LD excitation CW/Q-CW solid-state laser [wavelengths 488, 532, 635 nm], in view of the safety to the human body.

Further, examples of the fluorescence image acquisition means in the determination system may include a fluorescence microscope and confocal laser scanning microscopy. Examples of the fluorescence image acquired by such fluorescence image acquisition means may include the fluorescent still image and the autofluorescence image, and the fluorescence videograph as necessary.

Further, examples of the band pass filter for acquiring a fluorescence image in the determination system may include band pass filters for acquiring the fluorescent still image (the fluorescence videograph) and the autofluorescence image, and the band pass filter for acquiring a cell nucleus fluorescence image varies depending on the fluorescence properties of a nucleic acid staining agent to be used and, for example, since DAPI has the maximum fluorescence wavelength of 461 nm, Hoechst 33342 and Hoechst 33258 have the maximum fluorescence wavelength of 465 nm and PI (Propidium Iodide) has the maximum fluorescence wavelength of 617 nm, it is preferable to use a band pass filter which sufficiently covers these wavelengths.

Hereinafter, the present invention is described further in detail with reference to examples, but the technical scope of the present invention is not limited to the examples.

### Example 1

### [Method for preparing an immunostained tissue sample]

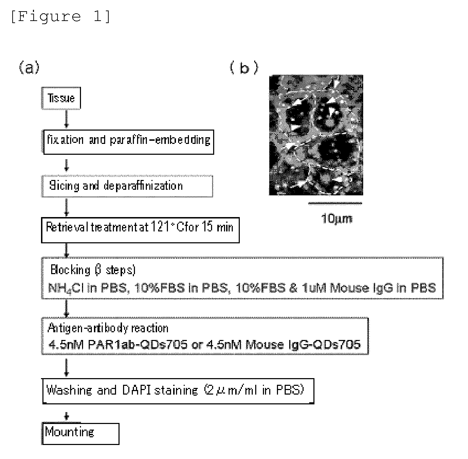

To differentiate from HercepTest (detecting breast cancer tissues using HER2 antibody), all tissue samples used as the human breast cancer pathological tissue were HercepTest negative. On the other hand, there are cancer cases which develop with no connection to three major breast cancer growth-related factors (ER, PgR, HER2) and this "triple negative" breast cancers are generally considered to have poor prognosis and to be difficult to treat. Under the circumstances, the "triple negative" breast cancer tissue samples were also studied. More specifically, the total of 16 samples consisting of 4 samples (one of which is the "triple negatives" [since 20 to 25% of the recurrent patients are the "triple negatives", 1 sample out of 4 samples was a randomly added "triple negative" sample]) of breast cancer tissues from recurrent patients (recurred within about 1 year and died within 4 years), 8 samples (including the above 1 sample) of "triple negative" breast cancer tissues from recurrent patients (recurred within 4 years and died within 6 years) and 5 samples of tissue from recurrence-free breast cancer survivors for at least 5 years were used, and tissue samples were prepared using these tissues by the method routinely used for the histopathological diagnosis. More specifically, cancer patient samples were fixed using formalin, dehydrated with alcohol, and then treated with xylene and immersed in paraffin at a high temperature for paraffin-embedding to prepare tissue samples (Figure 1(a)). Additionally, 4 samples of normal mammary tissues from cancer patients were used as the control. The tissues samples from the above 20 samples were cut to a 2 to 4 µm sections, deparaffinized using xylene, treated with alcohol and then washed with deionized water. To give effective access for the PAR1 antibody to the PAR1 antigen site in the fixed tissue, the above samples were retrieved in a 10 mM citric acid solution (pH 6.0) under the condition of 121°C for 15 minutes to loosen the fixed tissue structure. Subsequently, the above samples were washed in the order of deionized water and phosphate buffered saline (PBS), and the samples were then immersed in the order of in a 50 mM NH₄Cl/PBS solution at 25°C for 30 minutes, a 10%FBS/PBS solution at 25°C for 60 minutes and a 10%FBS + 1 µM mouse IgG/PBS solution at 25°C for 3 hours for the blocking treatment to prevent the nonspecific binding of the PAR1 antibody. Next, the above samples were subjected to an antibody reaction at 25°C for 1.5 hours in a solution (10%FBS + 1 µM mouse IgG/PBS) containing 4.5 nM of PAR1 antibody (PAR1ab-QD705) labeled with quantum dot fluorescent particles (Qdot705) emitting the 705 nm fluorescence. Additionally, mouse IgG labeled with quantum dot fluorescent particles (Qdot705) emitting the 705 nm fluorescence (Mouse IgG-QD705) was used as the control. After washing 4 times with a PBS solution, the DAPI staining (2 µg/ml PBS) was carried out at 25°C for 10 minutes and the cell nucleus was stained to count the number of cells. After washing 3 times with PBS, the cells were mounted and immunostained tissue samples were produced.

### Example 2

### [Problems in fluorescent immunohistostaining method]

The immunostained tissue samples produced in the above Example 1 were irradiated with 488 nm excitation light using a combination device of a confocal unit (a product of Yokogawa Electric Corporation), a fluorescence microscope (a product of OLYMPUS CORPORATION) and Electron-Multiplier CCD (EM-CCD) Camera (Andor Co., Ltd.), and then a fluorescence image (fluorescent still image) of quantum dot fluorescent particle (detected using PAR1ab-QD705) was acquired using a 695 to 740 nm band pass filter. Figure 1(b) shows the fluorescent still images of the tissue samples from recurrent breast cancer patients as an example, but the autofluorescence was so intense that the fluorescence of quantum dot fluorescent particle (detected using PAR1ab-QD705) was not identified on the first inspection. When the fluorescent brightnesses of autofluorescence and quantum dot fluorescent particles (detected using PAR1 antibody) were compared, only about 2 to 3 times difference was found in reality. Further, since the autofluorescence significantly varies depending on the type of a tissue sample and the place in a tissue sample, a definite threshold cannot be determined. Consequently, the autofluorescence need to be eliminated to accurately measure the fluorescent brightness of quantum dot fluorescent particle (detected using PAR1 antibody).

(a) Autofluorescence photobleaching method by excitation light irradiation, (b) contrast adjustment method and the like are known as methods for eliminating the influence of autofluorescence in the biological tissue staining (Figure 2). In the above (a) autofluorescence photobleaching method by excitation light irradiation, it is possible to bleach only the autofluorescence while the brightness intrinsic to quantum dot fluorescent particles is left unbleached by light irradiation for an extended period of time due to the light stability of quantum dot fluorescent particles, however, it poses drawbacks in that the complete bleaching of autofluorescence requires many hours and only autofluorescence at an irradiated area can be bleached. Further, in the above (b) contrast adjustment method, when the fluorescent brightness of quantum dot fluorescent particles (PAR1ab-QD705) is sufficiently stronger than the brightness of autofluorescence, it is possible to eliminate only the brightness of autofluorescence, however, when there is not enough difference between the brightnesses of quantum dot fluorescent particles (PAR1ab-QD705) and autofluorescence, the fluorescent brightness of quantum dot fluorescent particles is considered to diminish by the contrast adjustment. Further, a definite threshold cannot be determined because the autofluorescence widely varies depending on the kind of and place in a sample.

### Example 3

### [Production of corrected fluorescence image from which autofluorescence is eliminated]

A fluorescent still image with a zero (0) fluorescent intensity of the background autofluorescence need to be acquired. Then, the total fluorescence of fluorescent still image can be calculated as the fluorescence derived from quantum dot fluorescent particles. The contrast adjustment method of the above Example 2 uses the division of fluorescent intensity, thus failing to produce zero (0) by the division, and accordingly an image processing method which uses the subtraction capable of producing zero (0) is required. Under the circumstances, the following method is proposed as the image processing method for eliminating the autofluorescence of a fluorescent still image. In the method, first, a breast cancer tissue sample immunostained with quantum dot fluorescent particles is irradiated with excitation light (laser) having an excitation wavelength of 488 nm, an fluorescence image of quantum dot fluorescent particles (fluorescence of the quantum dot fluorescent particles + autofluorescence) is acquired using a band pass filter having an acquisition region of fluorescence wavelength of 695 to 740 nm, and then an autofluorescence image carrying only the autofluorescence is acquired in exactly the same focal plane and in the same field of vision as those of the acquired fluorescence image using a band pass filter having an acquisition region of fluorescence wavelength of 640 to 690 nm. Subsequently, the thus acquired fluorescence image (fluorescence of quantum dot fluorescent particles + autofluorescence) and the autofluorescence image are loaded in 512 x 512 pixels to convert to JPEG images in a 256-step gradation (Figure 3(a)), and then the brightness information (a value from 0 to 255) included in the autofluorescence image (autofluorescence) is subtracted from the brightness information (a value from 0 to 255) included in each pixel of the fluorescent still image (fluorescence of quantum dot fluorescent particles + autofluorescence) to produce an image showing only the fluorescence of quantum dot fluorescent particles (corrected fluorescence image).

To produce such a corrected fluorescence image, the fluorescence wavelength properties of autofluorescence in various wavelength ranges were examined. After irradiating cancer tissue samples, which were not immunostained with quantum dot fluorescent particles, with excitation light having an excitation wavelength of 488 nm, autofluorescence images in the same field of vision and the same focal point were acquired using 6 types of band pass filters in total having an acquisition region of fluorescence wavelength of 505 to 545 nm, 565 to 595 nm, 585 to 630 nm, 640 to 690 nm, 695 to 740 nm and 760 to 800 nm. A 512 x 512 pixel image in the same region of each of the autofluorescence images acquired using the 6 types of band pass filters was converted to a JPEG image, and in the JPEG image, a 512 x 420 pixel (total 210540 pixels) image in the same region was cut out to determine the mean value of fluorescent brightnesses in the image, thereby calculating "fluorescent brightness mean value of autofluorescence image / pixel" (value 1)). Further, in each of the autofluorescence images acquired by the above 6 types of band pass filters, an image having the background with no autofluorescence was acquired as the background fluorescence image, the fluorescent brightness of the acquired background fluorescence image was measured. In the measurement, the fluorescent brightness in the 25 x 25 pixel background fluorescence image was determined in any 3 regions, and "fluorescent brightness mean value of the background fluorescence image / pixel" was calculated (value 2)). These 3 regions measured were all same regions in each of the autofluorescence images acquired using the above 6 types of band pass filters. Using the values of the above 1) and 2), the fluorescent brightness mean value of autofluorescence from which the fluorescent brightness of background fluorescence was eliminated (hereinafter referred simply to as "fluorescent brightness of autofluorescence") was calculated based on the formula "(the fluorescent brightness mean value of autofluorescence image / pixel - the fluorescent brightness mean value of the background fluorescence image / pixel) x the number of pixel" = "(value 1) - value 2)) x the number of pixel". The fluorescent brightness of autofluorescence of each of the above 6 types of band pass filters was calculated, which was then divided by the fluorescence wavelength width acquired by each of the band pass filters (505 to 545 nm: 40 nm, 565 to 595 nm: 30 nm, 585 to 630 nm: 45 nm, 640 to 690 nm: 50 nm, 695 to 740 nm: 45 nm, 760 to 800 nm: 40 nm) to calculate the fluorescent brightness mean value of autofluorescence per wavelength width. The chart showing the fluorescence wavelength behavior of the autofluorescence was made by plotting the fluorescent brightness of the autofluorescence per such the wavelength in the center of the fluorescence wavelength acquired by each of the band pass filters, and connecting with a dot line (Figure 3(b)).

Next, to correspond the fluorescence wavelength behavior of quantum dot fluorescent particle to the fluorescence wavelength behavior of the above autofluorescence, the breast cancer tissue sample immunostained with quantum dot fluorescent particles by the method described in the above Example 1 was excited with the 488 nm excitation light, and a fluorescent still image (quantum dot fluorescent particle + autofluorescence) was acquired using a band pass filter having an acquisition region of fluorescence wavelength of 695 to 740 nm. Subsequently, a fluorescence videograph in the same field of vision and the same focal point as those of the acquired fluorescent still image (quantum dot fluorescent particle + autofluorescence) was acquired, and a single particle of the quantum dot fluorescent particles was specified by the method described in the following Example 4 (the method which uses the blinking reaction of the quantum dot fluorescent particle) using the fluorescence videograph. Thereafter, using the fluorescent still image, the fluorescent brightness of the specified single particle of the quantum dot fluorescent particles was measured. More specifically, targeting a single quantum dot fluorescent particle which does not lie on top of the autofluorescence, the fluorescent brightness of a pixel range (9 to 25 pixels) covering throughout the entire fluorescence of the single particle is determined and the "fluorescent brightness of fluorescent still image / pixel) was calculated (value 3)). Further, in the fluorescent still image, an image having the background with no autofluorescence or fluorescence of quantum dot fluorescent particles was acquired as the background fluorescence image, and the fluorescent brightness of the acquired background fluorescence image was measured. In the measurement, the fluorescent brightness of any 3 regions, which have the same number of pixel as that when the fluorescent brightness of the single quantum dot fluorescent particle was measured, was determined and "fluorescent brightness mean value of the background fluorescence image / pixel" was calculated (value 4)). Using the values of the above 3) and 4), the fluorescent brightness of quantum dot fluorescent particles from which the fluorescent brightness of background fluorescence was eliminated (hereinafter referred simply to as "fluorescent intensity of quantum dot fluorescent particle") was calculated based on the formula "(the fluorescent brightness mean value of fluorescent still image / pixel - the fluorescent brightness mean value of the background fluorescence image / pixel) x the number of pixel" = "(value 3) - value 4)) x the number of pixel". Further, in the fluorescent still image (quantum dot fluorescent particles + autofluorescence), the fluorescent brightness of any 3 regions, which have the same number pixel as that when the fluorescent brightness of the single quantum dot fluorescent particle was measured, was determined, from the region showing only the autofluorescence not including the fluorescence of the quantum dot fluorescent particle, and the "fluorescent brightness mean value of the autofluorescence images / pixel" was calculated (value 5)). Using the value 5), the fluorescent brightness of quantum dot fluorescent particles corresponding to the fluorescent brightness of autofluorescence was calculated based on the formula "the fluorescent brightness of quantum dot fluorescent particles / ([fluorescent brightness mean value of autofluorescence image / pixel] x the number of pixel)" = ([value 3) - value 4)] x the number of pixel) / ([value 5) - value 4) ] x the number of pixel). The above procedure was performed to 15 different fluorescent still images (quantum dot fluorescent particles + autofluorescence), the mean value (the fluorescent brightness mean value of quantum dot fluorescent particles to the fluorescent brightness of the autofluorescence) was determined from the values of the acquired 15 fluorescent particles in total and thereby calculated as 1.88 ± 0.10 (mean value ± s.e.m.). For the fluorescent brightness change of quantum dot fluorescent particles (Qdot705), an area representing the fluorescent brightnesses of the quantum dot fluorescent particles and autofluorescence was determined so that the (an area representing the fluorescent brightness of Qdot705) / (an area representing the fluorescent brightness of autofluorescence) ratio is 1.88, and a chart showing the fluorescence wavelength behaviors of the autofluorescence together with Qdot705 was produced (Figure 3(b)), with reference to, for example, the description in the pamphlet (file name: 2008-20-CBQdot) found in the Invitrogen website (http://www.invitrogen.jp/qdot/index.shtml). Additionally, such an area was determined by considering, for the sake of convenience, the area representing the fluorescent brightness of autofluorescence having a fluorescence wavelength of 695 to 740 nm as square. The (an area representing the fluorescent brightness of Qdot705) / (an area representing the fluorescent brightness of autofluorescence) ratio of 1.88 suggests that the fluorescent brightness of autofluorescence to be the background accounts for over 50% of the total fluorescent brightness (the fluorescent brightness of quantum dot fluorescent particles + the fluorescent brightness of autofluorescence) in the fluorescent still image, and it was reconfirmed that the fluorescent brightness of autofluorescence needs to be eliminated to quantify the fluorescent brightness of quantum dot fluorescent particles.

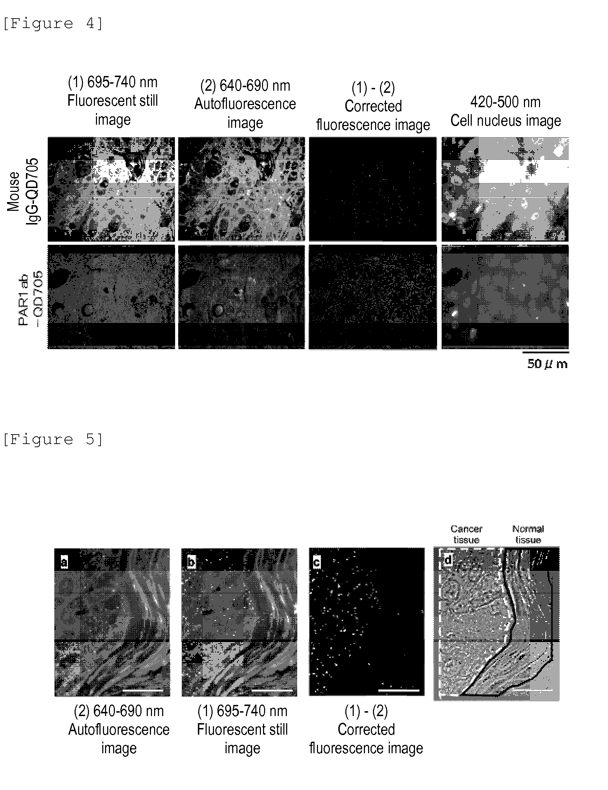

The corrected fluorescence image from which autofluorescence is eliminated was produced in accordance with the following procedure. More specifically, a tissue sample stained using PAR1ab-QD705 was irradiated with the 488 nm excitation light for 20 seconds, and a fluorescent still image (quantum dot fluorescent particles + autofluorescence) (20 s / frame x 1) acquired using a band pass filter having an acquisition region of fluorescence wavelength of 695 to 740 nm and an autofluorescence image (20 s / frame x 1) acquired in the same field of vision and the same focal point as those of the above fluorescent still image using a band pass filter having an acquisition region of fluorescence wavelength of 640 to 690 nm were acquired. Further, to count the number of cells, the tissue sample was excited with the 400 nm excitation light, and a cell nucleus image (detected using DAPI) acquired using a band pass filter having an acquisition region of fluorescence wavelength of 420 to 500 nm was acquired (Figure 4, first from right). The fluorescent still image (quantum dot fluorescent particle + autofluorescence) and the autofluorescence of the same region in the autofluorescence image were compared, and the maximum value of autofluorescence at any ROI (e.g., 25 x 25 pixels) was adjusted to be "fluorescent still image (quantum dot fluorescent particle + autofluorescence) x 1.2 times = autofluorescence image". More specifically, the autofluorescence image was adjusted to have an about 20% higher brightness value of autofluorescence than the fluorescent still image (quantum dot fluorescent particle + autofluorescence). Thus, with the brightness derived from the autofluorescence being adjusted, the images are converted to a JPEG image (Figure 4, first from left and second from left). Additionally, at the time of converting to a JPEG image, a threshold was set so that all fluorescence is imported. To eliminate the autofluorescence and acquire the fluorescent still image showing only quantum dot fluorescent particles, the treatment of "fluorescent still image (quantum dot fluorescent particle + autofluorescence) - autofluorescence image" was performed using a Photoshop image analysis software (Figure 4, second from right). At this treatment, it was confirmed in an optional plurality of regions that the brightness of a single quantum dot fluorescent particle is not diminished and the fluorescent intensity of the autofluorescence portion is zero (0) after the subtraction.

Since the autofluorescence of cancer tissue samples was effectively eliminated, subsequently whether the similar elimination of autofluorescence also from tissue samples, in which cancer cell tissues and normal cell tissues are present close to each other, is possible or not was examined. After confirming that cancer cell tissues can be specifically stained with quantum dot fluorescent particles (detected using PAR1ab-QD705) (Figure 5(b)), the image processing for eliminating the autofluorescence was carried out, and it was revealed that only the autofluorescence of cancer cell tissues and normal cell tissues were effectively eliminated, whereby a fluorescent still image showing only quantum dot fluorescent particles of cancer cell tissues (corrected fluorescence image) can be acquired (Figure 5(c)). This result suggests that a fluorescent still image for acquiring a corrected fluorescence image is not limited to the fluorescent still image showing cancer tissues throughout the entire field of vision as shown in Figure 4.

### Example 4

### [Calculation method of mean fluorescent brightness per single quantum dot fluorescent particle]

To calculate the mean fluorescent brightness per quantum dot fluorescent particle, whether a fluorescent brightness is derived from a single particle or not need to be determined and the "blinking property" distinctive of the quantum dot was used herein. Given 10% of the maximum fluorescent brightness of quantum dot fluorescent particles to be the threshold to be the limits of the blinking (see the arrowhead in Figure 6), a single quantum dot fluorescent particle has an off-state of about 4 seconds in 20 seconds of irradiation time (Figure 6). Accordingly, a single particle fluorescent brightness can be acquired when the mean intensity of particles having an about 4 second-off-state is measured. Thus, to acquire a fluorescence videograph corresponding to a fluorescent still image, a 100 consecutive series of fluorescent still image in 200 msec of resolution after irradiated with excitation light was acquired using a band pass filter having an acquisition region of fluorescence wavelength of 690 to 730 nm, and a fluorescence videograph (200 ms/frame x 100 images) was acquired. Using the above fluorescence videograph, a particle having an off-state of about 4 seconds was identified as a single fluorescent particle and then the fluorescent brightness of the corresponding particle in the still image was determined using an Image J image analysis software. This procedure was carried out using 5 to 10 particles, and the mean fluorescent brightness per particle was calculated.

### Example 5

### [Method for calculating the number of particles per cell]

The number of quantum dot fluorescent particles per cell can be calculated by inputting the value of total fluorescent brightness of the quantum dot fluorescent particles acquired from the corrected fluorescence image (the total fluorescent brightness in the image), the number of cells acquired from the cell nucleus stained image (detected using DAPI), and further, the value of mean fluorescent brightness per quantum dot fluorescent particle (a single particle fluorescent brightness) into the formula [(the total fluorescent brightness in the image / the single particle fluorescent brightness) / the number of cells = the total number of fluorescent particles in the image / the number of cells = the number of fluorescent particles / cell]. Furthermore, the same procedure was performed in the case where mouse IgG labeled with quantum dot fluorescent particle which is the control was used, "the number of quantum dot fluorescent particles detected using the PAR1 antibody / cell - the number of quantum dot fluorescent particles detected using mouse IgG / cell" was calculated to subtract the number of nonspecific particles from the number of quantum dot fluorescent particles (detected using the PAR1 antibody), whereby the true number of fluorescent particles per cell detected using the PAR1 antibody was calculated. The calculation of fluorescent particle numbers was performed for each sample (Sample 1 to 20) shown in Table 1, using 300 to 1000 cells respectively, and the mean value of true number of particles detected using the PAR1 antibody was determined. The results are shown in Table 1 (under "PAR1ab-QDs score").

**[Table 1]**

| | Recurrence | Sample No. | Age | Cancer stage | HER2 score | ER/PgR | Metastasis site | Recurrence-free survival period | PAR1ab-QDs score |
|---|---|---|---|---|---|---|---|---|---|
| Normal tissues | | Sample 1 | 35 | | | | | | 0.3 |
| | | Sample 2 | 47 | | | | | | 0.1 |
| | | Sample 3 | 55 | | | | | | 0.1 |
| | | Sample 4 | 56 | | | | | | 0.2 |
| Cancer tissues | Tissues from patients free of recurrence for at least 5 years | Sample 5 | 56 | IIIA | 0 | +/+ | | | 4.8 |
| | | Sample 6 | 49 | IA | 1 | +/+ | | | 5.1 |
| | | Sample 7 | 62 | IIB | 1 | +/+ | | | 2.5 |
| | | Sample 8 | 41 | IIB | 1 | +/+ | | | 2.1 |
| | | Sample 9 | 58 | IIB | 0 | +/+ | | | 1.3 |
| | Tissues from patients recurred within 4 years | Sample 10 | 35 | IIA | 0 | +/- | Lung | 0.83 | 13 |
| | | Sample 11 | 47 | IIIC | 0 | +/+ | Bone, Liver | 1.08 | 6.3 |
| | | Sample 12 | 51 | IA | 0 | -/+ | Brain, Lung, Lymph node, | 0.83 | 25.2 |
| | | Sample 13* | 40 | IIB | 1 | -/- | Brain, Lung, | 0.83 | 12.2 |
| | | Sample 14* | 54 | IIA | 0 | -/- | Brain, Liver, Lung | 0.75 | 1.7 |
| | | Sample 15* | 60 | IIIC | 1 | -/- | Bone, Liver, lymph node, | 2 | 0.5 |
| | | Sample 16* | 42 | IIB | 1 | -/- | Brain, Lung | 2 | 4 |
| | | Sample 17* | 80 | IA | 0 | -/- | Liver, Lung | 0.67 | 5.5 |
| | | Sample 18* | 75 | IIIC | 0 | -/- | Bone, Liver | 1.75 | 0.5 |
| | | Sample 19* | 41 | IA | 1 | -/- | Bone, Liver | 2.58 | 0.5 |
| | | Sample 20* | 55 | IIIC | 0 | -/- | Brain, Liver, Lung | 0.83 | 23.2 |

| | | | | | | | | | |
|---|---|---|---|---|---|---|---|---|---|
| * indicates triple negative samples (Samples 13 to 20) | | | | | | | | | |

### Example 6

### [Calculation of the number of particles detected using the PAR1 antibody per breast cancer tissue cell]

The true number of particles detected using the PAR1 antibody (under "PAR1ab-QDs score" in Table 1) had the mean value of 14.8 in 3 samples (see Samples 10 to 12, Table 1) of breast cancer tissues from recurrent patients (recurred within about 1 year and died within 4 years) which was higher than the mean value of 3.2 in 5 samples (see Samples 5 to 9, Table 1) of tissues from recurrence-free breast cancer survivors for at least 5 years, on the other hand, 4 samples (see Samples 1 to 4, Table 1) of normal mammary tissues from cancer patients had the mean value of 0.2, which was significantly lower than that of the breast cancer tissues (Table 1, Figure 7(a)). Furthermore, to calculate the number of particles in the "triple negative" breast cancer tissues, the mean number of particle for 8 samples (see Samples 13 to 20, Table 1) of "triple negative" breast cancer tissues from recurrent patients (recurred within 4 years and died within 6 years) were calculated and found to have 6.0, which was higher value than that for normal mammary tissues from cancer patients and tissues from recurrence-free breast cancer survivors for at least 5 years (Table 1, Figure 7(a)). The "triple negatives" is a serious issue for which Herceptin is not valid, and thus the immunohistostaining method which uses the PAR1 antibody of the present invention has the potential to be a breakthrough therein. Further, based on the breast cancer tissue samples from recurrent patients (see Samples 10 to 20, Table 1), the relevance of the true particle numbers detected using the PAR1 antibody to the number of year before breast cancer recurs was studied and revealed, with strong relevance, that the larger number of true particles detected using the PAR1 antibody has the shorter period before the breast cancer recurrence (Figure 7(b)) (correlation coefficient (R) = 0.75). Then, the "triple negative" breast cancer tissues from recurrent patients were specifically studied but the same result was obtained (Figure 7(c)) (correlation coefficient (R) = 0.71). These results suggest that the immunohistostaining method using the PAR1 antibody of the present invention has the potential capable of predicting the recurrence interval of breast cancers including the "triple negatives".

### Example 7

### [Examination on excitation wavelength conditions]

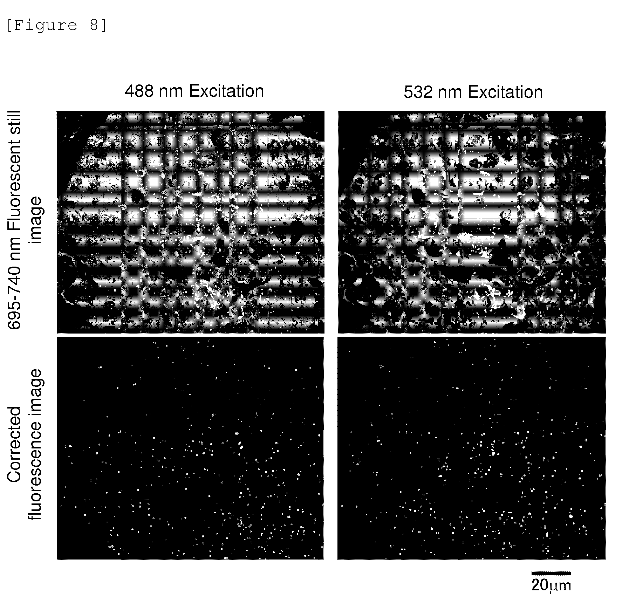

Furthermore, the applicability of the excitation wavelength of excitation light other than 488 nm was studied using an excitation wavelength of 532 nm, and found that the number of particles detected using the PAR1 antibody per cell of Sample 13 tissue sample was 15.5 (the number of particles / the number of cells) with an excitation wavelength of 488 nm, whereas 15.8 (the number of particles / the number of cells) with the wavelength of 532 nm (Figure 8), with the number of particles detected using the PAR1 antibody per cell calculated in both wavelengths being no notable difference (Figure 8). This result suggests that the wavelength of excitation light applicable is not limited to 488 nm.

### Example 8

### [Examination on the wavelength property eliminating autofluorescence wavelength]

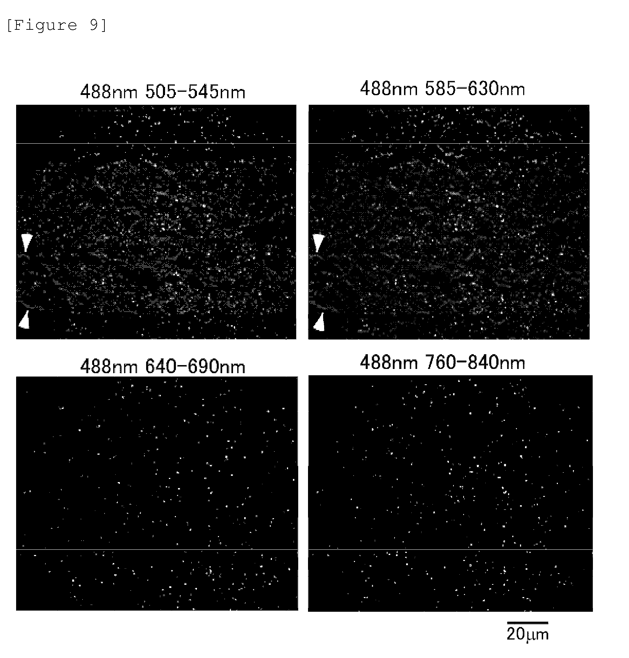

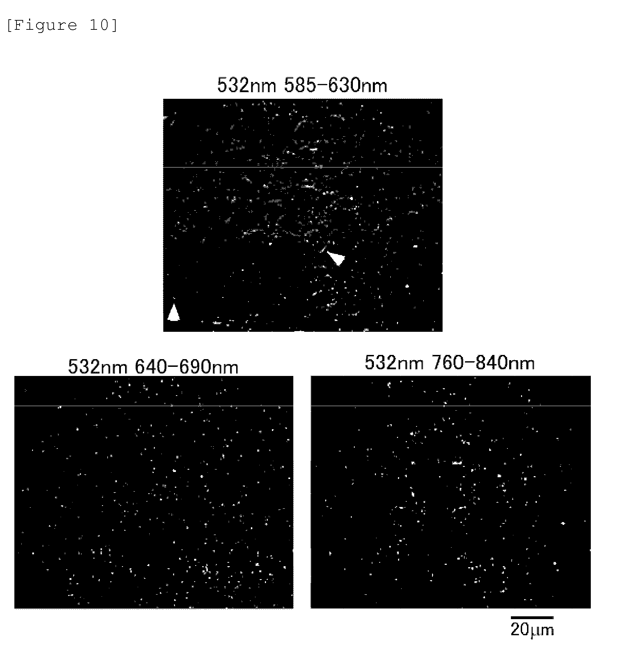

Further, for the wavelength range of the band pass filter for acquiring an autofluorescence image, the band pass filter having an acquisition region of fluorescence wavelength of 505 to 545 nm, 585 to 630 nm, 640 to 690 nm or 760 to 840 nm in addition to the band pass filter having an acquisition region of fluorescence wavelength of 640 to 690 nm when excitation light has an excitation wavelength of 488 nm (Figure 9) and the band pass filter having an acquisition region of fluorescence wavelength of 585 to 630 nm, 640 to 690 nm or 760 to 840 nm in addition to the band pass filter having an acquisition region of fluorescence wavelength of 640 to 690 nm when excitation light has an excitation wavelength of 532 nm (Figure 10) were studied. In both 488 nm and 532 nm excitation lights, the autofluorescence remained as indicated by the arrowheads when a corrected fluorescence image was acquired based on the autofluorescence image acquired in a wavelength range on the shorter wavelength side than the band pass filter having an acquisition region of fluorescence wavelength of 635 to 685 nm. This is because that when an autofluorescence image is acquired in a wavelength range apart from the wavelength range in which a fluorescent still image is acquired, the autofluorescence out of the focal plane is detected and the autofluorescence out of the focal plane still remains after the autofluorescence subtraction. Conversely, when a corrected fluorescence image is acquired based on the autofluorescence image acquired at 760 to 840 nm on the long wavelength side, the autofluorescence is subtractable but the fluorescence accompanying the out-of-focal plane was found on the image. The reason why the defocus was less found than in the band pass filters having an acquisition region of fluorescence wavelength of 505 to 545 nm or 586 to 630 nm is because it is assumed that the sensitivity of camera used suddenly drops at 800 nm and thus when a band pass filter having an acquisition region of fluorescence wavelength of 760 to 840 nm is used, the autofluorescence actually detected had "760 to 800 nm + slightly more than 800 nm". In conclusion, the autofluorescence either on the short wavelength side or long wavelength side of the fluorescence wavelength of fluorescence particles is subtractable, but, due to the chromatic aberration problem, it is conceived that the subtraction is better performed at a vicinity wavelength of fluorescence wavelength of fluorescence particles.

### Example 9

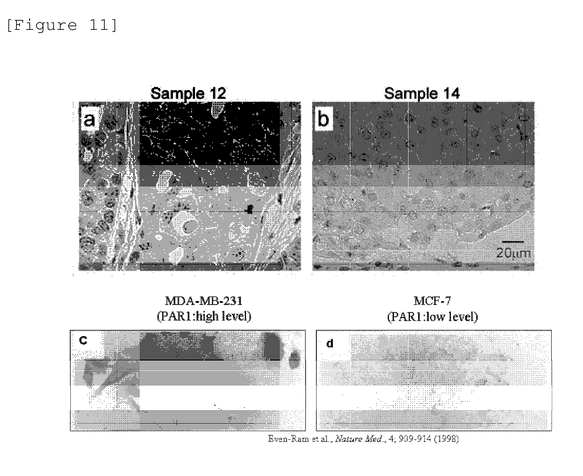

The immunohistostaining method for calculating the number of quantum dot particles according to the present invention was studied to find any actual difference compared with the DAB staining method by performing a comparative experiment with the DAB staining method. Sample 12 tissue sample and Sample 14 tissue sample shown in Table 1 were subjected to the treatment steps up to the deparaffinization by the method described in the above Example 1, and then washed with deionized water. To remove the endogenous peroxidase in the tissue samples, the samples were treated with a methanol solution containing 30% hydrogen peroxide solution (H₂O₂) for 10 minutes, and then washed 3 times with distilled water. To give an effective access for the PAR1 antibody to the PAR antigen site in the fixed tissue, the above samples were retrieved in a 10 mM citric acid solution (pH 6.0) under the conditions of 121°C for 15 minutes to loosen the fixed tissue structure. Subsequently, the above samples were washed in the order of deionized water and phosphate buffered saline (PBS), and then the samples were treated in a 50 mM NH₄Cl/PBS solution at 25°C for 30 minutes, further treated in a 10%FBS/PBS solution at 25°C for 2 hours for the blocking treatment to prevent the nonspecific binding of the PAR1 antibody. Next, the samples were subjected to a primary antibody reaction in a 10%FBS/PBS solution containing 33nM PAR1 antibody at 25°C for 2 hours. After washing 4 times with the PBS solution, a secondary antibody reaction was performed in a 10%FBS/PBS solution containing 22nM secondary antibodies to mouse antibody at 25°C for 1 hour. After washing 4 times with the PBS solution, the DAB luminescence was carried out at a color developing time of 25°C for 5 minutes, using a peroxidase stain DAB kit (a product of NACALAI TESQUE, INC.). After washing the tissue samples with tap water, hematoxylin staining was carried out. After washing the tissue samples with tap water, the tissue samples were penetrated, dehydrated and mounted to complete the DAB-stained tissue samples, and the observation results by a microscope are shown in Figure 10. In Sample 12 tissue sample which had the large number of quantum dot particles, the nuclei, cytoplasms and membranes were found to have been stained (normal cells have only nucleus stained with hematoxylin) (Figure 11(a)). On the other hand, in Sample 14 tissue sample which had fewer number of quantum dot particles, there was not much specific staining (Figure 11(b)). Also, as shown in Even-Ram et al., Nature Med., 4; 909-914 (1998), when breast cancer cells were stained by the DAB method using the PAR1 antibody (Figures 11(c) and (d)), the high level expression line of PAR1 has the nucleus thereof stained, i.e., the nonspecific staining caused by the DAB method has been a problem. On the other hand, when the tissue staining method of the present invention is used, breast cancer cells can be stained in correlation with the DAB method, and breast cancer cells can be quantitatively specified with high accuracy in comparison with the DAB method of the conventional technology. In the tissue staining method of the present invention, the nonspecific PAR1 signal is not substantially detected in the cancer cell nucleus, and this presumably owes to the technical feature of the present invention which makes it possible to suitably eliminate the autofluorescence and specify the true quantum dot fluorescent particle using the blinking property.

### Industrial Applicability

The determination method of the present invention is valid when, for example, the PAR1 antibody which detects the breast cancer marker factor is used as the antibody labeled with quantum dot fluorescent particles, the number of fluorescent particles per cell detected using the PAR1 antibody is determined, and, using the number as the indicator, the pathological diagnosis of breast cancer onset or breast cancer onset risk is performed.

## Claims

1. A method for determining cancer onset or cancer onset risk, comprising using a tissue staining method comprising the following steps (a) to (d):
(a) labeling an antibody which recognizes a cancer growth regulatory factor or cancer metastasis regulatory factor with a fluorescent material, and contacting the fluorescent-labeled antibody with a tissue sample;
(b) irradiating a tissue site in contact with the antibody with excitation light of a given wavelength;
(c1) acquiring from step (b) a fluorescence image in an acquisition region of fluorescence wavelength emitted by the fluorescent material;
(c2) acquiring from step (b) an autofluorescence image in a vicinity region of a short wavelength side or long wavelength side of an acquisition region of fluorescence wavelength emitted by the fluorescent material, in the same field of vision and in the same focal point as those of the fluorescence image;
(d) acquiring a corrected fluorescence image by image processing to eliminate a fluorescent brightness of the autofluorescence image from a fluorescent brightness of the fluorescence image.

2. The determination method according to claim 1, wherein the cancer growth regulatory factor or cancer metastasis regulatory factor is PAR1 (protease activated receptor 1).

3. The determination method according to claim 1 or 2, wherein the cancer is a breast cancer.

4. The determination method according to any one of claims 1 to 3, wherein a difference in the wavelengths between the acquisition region of fluorescence wavelength emitted by the fluorescent material and the vicinity region thereof is within 100 nm.

5. The determination method according to any one of claims 1 to 4, wherein the acquisition region of fluorescence wavelength emitted by the fluorescent material is a near infrared region.

6. The determination method according to any one of claims 1 to 5, wherein the wavelength of the excitation light is 400 to 700 nm.

7. The determination method according to any one of claims 1 to 6, wherein the fluorescent material is a fluorescent particle.

8. The determination method according to claim 7, wherein the fluorescent particle is a quantum dot fluorescent particle.

9. The determination method according to claim 7 or 8, further comprising the following steps (e) to (g):
(e) counting the number of cells of the tissue site in contact with the antibody;
(f) identifying a single fluorescent particle based on the fluorescence image, and measuring a mean fluorescent brightness of a single fluorescent particle in the corrected fluorescence image corresponding to the single fluorescent particle; and
(g) calculating the number of fluorescent particles per cell by dividing a total fluorescent brightness in the corrected fluorescence image by the mean fluorescent brightness of the single fluorescent particle to determine the number of fluorescent particles, and dividing the number of fluorescent particles by the number of cells counted in the step (e).

10. The determination method according to claim 9, wherein the single fluorescent particle is identified using a blinking property of the fluorescent particles.

## Patentansprüche

1. Verfahren zum Bestimmen des Ausbrechens von Krebs oder des Risikos des Ausbrechens von Krebs, umfassend das Verwenden eines Gewebefärbeverfahrens, das die folgenden Schritte (a) bis (d) umfasst:
(a) Markieren eines Antikörpers, der einen Krebswachstums-Regulationsfaktor oder einen Krebsmetastase-Regulationsfaktor erkennt, mit einem fluoreszierenden Material und Inkontaktbringen des fluoreszenzmarkierten Antikörpers mit einer Gewebeprobe;
(b) Bestrahlen eines Gewebeorts in Kontakt mit dem Antikörper mit Anregungslicht mit einer gegebenen Wellenlänge;
(c1) Aufnehmen eines Fluoreszenzbilds von Schritt (b) in einem Aufnahmebereich mit der von dem fluoreszierenden Material emittierten Fluoreszenzwellenlänge;
(c2) Aufnehmen eines Autofluoreszenzbilds von Schritt (b) in einem benachbarten Bereich an einer Kurzwellenlängenseite oder Langwellenlängenseite eines Aufnahmebereichs mit der von dem fluoreszierenden Material emittierten Fluoreszenzwellenlänge in dem gleichen Sehfeld und mit dem gleichen Brennpunkt wie das Fluoreszenzbild;
(d) Aufnehmen eines korrigierten Fluoreszenzbilds durch Bildverarbeitung zum Entfernen von Fluoreszenzhelligkeit des Autofluoreszenzbilds von der Fluoreszenzhelligkeit des Fluoreszenzbilds.

2. Bestimmungsverfahren gemäß Anspruch 1, wobei der Krebswachstums-Regulationsfaktor oder Krebsmetastase-Regulationsfaktor PAR1 (proteaseaktivierter Rezeptor 1) ist.

3. Bestimmungsverfahren gemäß Anspruch 1 oder 2, wobei der Krebs ein Brustkrebs ist.

4. Bestimmungsverfahren gemäß einem der Ansprüche 1 bis 3, wobei der Unterschied der Wellenlängen zwischen dem Aufnahmebereich mit der von dem fluoreszierenden Material emittierten Fluoreszenzwellenlänge und dem dazu benachbarten Bereich innerhalb von 100 nm liegt.

5. Bestimmungsverfahren gemäß einem der Ansprüche 1 bis 4, wobei der Aufnahmebereich mit der von dem fluoreszierenden Material emittierten Fluoreszenzwellenlänge ein Nahinfrarotbereich ist.

6. Bestimmungsverfahren gemäß einem der Ansprüche 1 bis 5, wobei die Wellenlänge des Anregungslichts 400 bis 700 nm beträgt.

7. Bestimmungsverfahren gemäß einem der Ansprüche 1 bis 6, wobei das fluoreszierende Material ein fluoreszierendes Partikel ist.

8. Bestimmungsverfahren gemäß Anspruch 7, wobei das fluoreszierende Partikel ein fluoreszierendes Quantenpunkt-Partikel ist.

9. Bestimmungsverfahren gemäß Anspruch 7 oder 8, ferner umfassend die folgenden Schritte (e) bis (g):
(e) Zählen der Anzahl von Zellen des Gewebeorts in Kontakt mit dem Antikörper;
(f) Identifizieren eines einzelnen fluoreszierenden Partikels auf der Grundlage des Fluoreszenzbilds und Messen einer mittleren Fluoreszenzhelligkeit eines einzelnen fluoreszierenden Partikels in dem korrigierten Fluoreszenzbild, das dem einzelnen fluoreszierenden Partikel entspricht; und
(g) Berechnen der Anzahl von fluoreszierenden Partikeln pro Zelle durch Teilen der Gesamtfluoreszenzhelligkeit in dem korrigierten Fluoreszenzbild durch die mittlere Fluoreszenzhelligkeit des einzelnen fluoreszierenden Partikels, um die Anzahl von fluoreszierenden Partikel zu bestimmen, und Teilen der Anzahl von fluoreszierenden Partikel durch die bei Schritt (e) gezählte Anzahl von Zellen.

10. Bestimmungsverfahren gemäß Anspruch 9, wobei das einzelne fluoreszierende Partikel unter Verwendung einer Blinkeigenschaft der fluoreszierenden Partikel identifiziert wird.

## Revendications

1. Procédé de détermination de l'apparition d'un cancer ou du risque d'apparition d'un cancer, comprenant l'utilisation d'un procédé de coloration de tissu comprenant les étapes (a) à (d) suivantes :
(a) marquer un anticorps qui reconnaît un facteur de régulation de la croissance d'un cancer ou un facteur de régulation de la métastase d'un cancer avec un matériau fluorescent, et mettre l'anticorps marqué par fluorescence en contact avec un prélèvement tissulaire ;
(b) irradier un site tissulaire en contact avec l'anticorps avec une lumière d'excitation d'une longueur d'onde donnée ;
(c1) acquérir à partir de l'étape (b) une image de fluorescence dans une région d'acquisition de longueur d'onde de fluorescence émise par le matériau fluorescent ;
(c2) acquérir à partir de l'étape (b) une image d'autofluorescence dans une région voisine d'un côté des longueurs d'onde courtes ou d'un côté des longueurs d'onde longues d'une région d'acquisition de longueur d'onde de fluorescence émise par le matériau fluorescent, dans le même champ de vision et au même point focal que ceux de l'image de fluorescence ;
(d) acquérir une image de fluorescence corrigée par traitement d'image pour éliminer une intensité de fluorescence de l'image d'autofluorescence d'une intensité de fluorescence de l'image de fluorescence.

2. Procédé de détermination selon la revendication 1, dans lequel le facteur de régulation de la croissance du cancer ou le facteur de régulation de la métastase du cancer est le PAR1 (récepteur activé par les protéases 1).

3. Procédé de détermination selon la revendication 1 ou 2, dans lequel le cancer est un cancer du sein.

4. Procédé de détermination selon l'une quelconque des revendications 1 à 3, dans lequel une différence de longueur d'onde entre la région d'acquisition de longueur d'onde de fluorescence émise par le matériau fluorescent et la région voisine de celle-ci ne dépasse pas 100 nm.

5. Procédé de détermination selon l'une quelconque des revendications 1 à 4, dans lequel la région d'acquisition de longueur d'onde de fluorescence émise par le matériau fluorescent est une région dans le proche infrarouge.

6. Procédé de détermination selon l'une quelconque des revendications 1 à 5, dans lequel la longueur d'onde de la lumière d'excitation est de 400 à 700 nm.

7. Procédé de détermination selon l'une quelconque des revendications 1 à 6, dans lequel le matériau fluorescent est une particule fluorescente.

8. Procédé de détermination selon la revendication 7, dans lequel la particule fluorescente est une particule fluorescente quantique.

9. Procédé de détermination selon la revendication 7 ou 8, comprenant en outre les étapes (e) à (g) suivantes :
(e) compter le nombre de cellules du site tissulaire en contact avec l'anticorps ;
(f) identifier une particule fluorescente individuelle sur la base de l'image de fluorescence, et mesurer une intensité de fluorescence moyenne d'une particule fluorescente individuelle dans l'image de fluorescence corrigée correspondant à la particule fluorescente individuelle ; et
(g) calculer le nombre de particules fluorescentes par cellule en divisant une intensité de fluorescence totale dans l'image de fluorescence corrigée par l'intensité de fluorescence moyenne de la particule fluorescente individuelle pour déterminer le nombre de particules fluorescentes, et en divisant le nombre de particules fluorescentes par le nombre de cellules comptées à l'étape (e).

10. Procédé de détermination selon la revendication 9, dans lequel la particule fluorescente individuelle est identifiée en utilisant une propriété de clignotement des particules fluorescentes.
